# EUROPEAN PATENT APPLICATION

(11) **EP 4 674 958 A1**
(43) Date of publication of application: **07.01.2026**
(21) Application number: 24785350.0
(22) Date of filing: 05.04.2024
(51) Int. Cl.: C12N 9/90, C12N 15/77, C12P 13/14, C12P 13/10

(54) **MUTANT PROLYL ISOMERASE, AND GLUTAMATE-BASED AMINO ACID PRODUCTION METHOD USING SAME**

(30) Priority: 07.04.2023 KR 20230046163
(71) Applicant: CJ Cheiljedang Corporation, Seoul 04560 (KR)
(72) Inventor: PARK, So-Yeon, Seoul 04560 (KR); LEE, Ji Yeon, Seoul 04560 (KR); BYUN, Hyo Jeong, Seoul 04560 (KR); HAN, Seunghee, Seoul 04560 (KR)
(74) Representative: Meissner Bolte Partnerschaft mbB
(86) International application number: PCT/KR2024/004573
(87) International publication number: WO 2024/210653

(57) **Abstract**

The present application relates to a mutant prolyl isomerase, and a glutamic acid-based amino acid production method using same.

## Description

### [Technical Field]

The present application relates to a mutant prolyl isomerase, and a glutamic acid-based amino acid production method using same.

### [Background Art]

Glutamic acid is one of the proteinogenic amino acids widely found in plants, animals, and microorganisms, and is metabolized in the organs of the body into L-ornithine, L-citrulline, L-arginine, L-glutamine, *etc.*

Glutamic acid has a unique taste, and thus is widely used in pharmaceuticals and other animal feed industries, *etc.,* as well as in food industry. Additionally, L-ornithine has been used as nutritional supplements because it is effective in building muscles and reducing body fat, and also as medicine to improve cirrhosis and liver dysfunction. It is known that L-citrulline has physiological effects such as promoting ammonia metabolism, improving blood flow by vasodilation, lowering blood pressure, enabling neurotransmission, enhancing immunity, scavenging active oxygen, *etc.* Further, L-arginine has been widely used in medicine as hepatic function-promoting agents, brain function-promoting agents, and ingredients of multiple amino acid supplements, and also in the food industry as additives for fish cakes and health beverages, salt substitutes for patients with hypertension, *etc.*

Enhancement of the biosynthesis pathway of glutamic acid can promote not only glutamic acid, but also glutamic acid-based amino acids such as L-ornithine, L-citrulline, L-arginine, and L-glutamine. Methods for producing such glutamic acid include a method of producing glutamic acid through fermentation using coryneform bacteria including microorganisms belonging to the genus *Brevibacterium* or *Corynebacterium* and mutants thereof (Amino Acid Fermentation, Gakkai Shuppan Center: 195-215, 1986), and other methods using *Escherichia coli* and microorganisms belonging to the genus *Bacillus, Streptomyces, Penicillium, Klebsiella, Erwinia,* and *Pantoea* (US Patent No. 6,682,912), *etc.*

### [Disclosure]

### [Technical Problem]

The present disclosure relates to a mutant prolyl isomerase and a method for producing glutamic acid-based amino acids using the same.

### [Technical Solution]

One object of the present disclosure is to provide a mutant prolyl isomerase comprising an amino acid substitution at a position corresponding to position 95 of the amino acid sequence of SEQ ID NO: 1 with another amino acid and having at least 90% sequence identity to the amino acid sequence of SEQ ID NO: 1.

Another object of the present disclosure is to provide a polynucleotide encoding the mutant prolyl isomerase of the present disclosure.

Still another object of the present disclosure is to provide a microorganism including the mutant prolyl isomerase of the present disclosure; or a polynucleotide encoding the same.

Still another object of the present disclosure is to provide a method for producing glutamic acid-based amino acids, comprising: culturing a microorganism including the mutant prolyl isomerase of the present disclosure; or a polynucleotide encoding the same, in a medium.

### [Advantageous Effects]

When culturing a microorganism for producing glutamic acid-based amino acids using the mutant prolyl isomerase of the present disclosure, the production of glutamic acid-based amino acids, including ornithine, citrulline, arginine, and glutamine, increases compared to a microorganism having a conventional wild-type prolyl isomerase.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Additionally, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

One aspect of the present disclosure provides a mutant prolyl isomerase comprising an amino acid substitution at a position corresponding to position 95 of the amino acid sequence of SEQ ID NO: 1 with another amino acid and having at least 90% sequence identity to the amino acid sequence of SEQ ID NO: 1.

As used herein, the term "mutant prolyl isomerase" refers to a variant including a substitution of an amino acid corresponding to position 95 from the N-terminus of SEQ ID NO: 1 with another amino acid, in any polypeptide or prolyl isomerase having the function of regulating prolyl isomerase activity. That is, in the mutant prolyl isomerase in which the amino acid corresponding to position 95 in the amino acid sequence of SEQ ID NO: 1 of the present disclosure is substituted with another amino acid, SEQ ID NO: 1 is a reference sequence, which is a sequence used to determine the position of the amino acid in the amino acid sequence of any polypeptide or prolyl isomerase having prolyl isomerase activity. The "mutant prolyl isomerase" may be referred to as "prolyl isomerase variant", "fkpA variant", "mutant fkpA", "FkpA variant", "mutant FkpA", "PPlase variant", "mutant PPlase, *etc.*

The protein targeted for the introduction of mutation in the present disclosure may be a protein having prolyl isomerase activity. Specifically, the protein may include an amino acid sequence of SEQ ID NO: 1 and have prolyl isomerase activity, but it is not limited thereto. The protein does not exclude the addition of a meaningless sequence upstream or downstream of the amino acid sequence of SEQ ID NO: 1, a naturally occurring mutation, or a silent mutation therein, and any protein may fall within the scope of the protein targeted for the introduction of mutation in the present disclosure as long as the protein has activity identical to or corresponding to the activity of the protein including the amino acid sequence of SEQ ID NO: 1. For example, the protein targeted for the introduction of mutation in the present disclosure may be a protein including the amino acid sequence of SEQ ID NO: 1 or an amino acid sequence having a homology or identity of 80%, 85%, 90%, 95%, 96%, 97%, 98%, or 99% or higher thereto, or it may be a protein consisting of or consisting essentially of an amino acid sequence having the above homology or identity. Additionally, it is apparent that any protein having an amino acid sequence, in which part of the sequence is deleted, modified, substituted, or added, may also fall within the scope of the protein targeted for mutation in the present disclosure as long as the amino acid sequence has such a homology or identity, and exhibits an efficacy corresponding to the protein.

As used herein, the term "prolyl isomerase (prolyl cis/trans isomerase)" refers to an enzyme expressed as a cluster with citrate synthase (CS) in microorganisms, and is an enzyme that delays the aggregation of citrate synthase. Additionally, it is known to have an effective chaperone function via the N-terminal domain of prolyl isomerase having a chaperone function in microorganisms under high-temperature conditions. The prolyl isomerase may be used interchangeably with "peptidyl-prolyl cis-trans isomerase", "FKBP (FK506 binding protein) type peptidyl-prolyl cis-trans isomerase", "FkpA" or "PPlase". The amino acid sequence of FkpA can be obtained from a known database, such as NCBI's Genebank.

In one example, the FkpA protein may be derived from a microorganism, specifically derived from a prokaryotic microorganism or a eukaryotic microorganism, and more specifically derived from a microorganism of the genus *Corynebacterium, etc.,* but it is not limited thereto

In another example, the FkpA protein may be NCgl0796(Cg0950) derived from a microorganism of the genus *Corynebacterium,* but it is apparent that any protein having prolyl isomerase activity of various origins may be included.

In the present disclosure, the amino acid before modification corresponding to position 95 of SEQ ID NO: 1 in the amino acid sequence before modification of the FkpA protein targeted for modification may be tyrosine (Y).

The mutant prolyl isomerase of the present disclosure may be one in which the amino acid corresponding to position 95 in the amino acid sequence of SEQ ID NO: 1 is substituted with an amino acid different from the amino acid before substitution.

In one example, the mutant prolyl isomerase may be one in which the amino acid corresponding to position 95 in the amino acid sequence of SEQ ID NO: 1 is substituted with any one or more amino acids selected from the group consisting of methionine, serine, threonine, asparagine, cysteine, histidine, lysine, aspartic acid, alanine, valine, leucine, glutamine, glycine, proline, glutamic acid, arginine, isoleucine, phenylalanine, and tryptophan. In another example, the mutant prolyl isomerase may be one in which the amino acid corresponding to position 95 in the amino acid sequence of SEQ ID NO: 1 is substituted with any one or more amino acids selected from the group consisting of methionine, serine, threonine, asparagine, cysteine, histidine, lysine, aspartic acid, alanine, valine, leucine, glutamine, glycine, proline, and glutamic acid. In still another example, the mutant prolyl isomerase may be one in which the amino acid corresponding to position 95 in the amino acid sequence of SEQ ID NO: 1 is substituted with an amino acid other than an aromatic amino acid. In still another example, the mutant prolyl isomerase may be one in which the amino acid corresponding to position 95 in the amino acid sequence of SEQ ID NO: 1 is substituted with an amino acid having a nonpolar, polar, or charged side chain.

In still another example, the mutant prolyl isomerase of the present disclosure may have, include, consist of, or consist essentially of an amino acid sequence represented by SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, or SEQ ID NO: 31.

In one example, the mutant prolyl isomerase of the present disclosure may be one in which the amino acid corresponding to position 95 in the amino acid sequence of SEQ ID NO: 1 is substituted with an amino acid other than tyrosine.

The mutant prolyl isomerase of the present disclosure may be one in which the amino acid corresponding to position 95, based on the amino acid sequence of SEQ ID NO: 1 is an amino acid other than tyrosine, for example, methionine, serine, threonine, asparagine, cysteine, histidine, lysine, aspartic acid, alanine, valine, leucine, glutamine, glycine, proline or glutamic acid, and may include an amino acid sequence having a homology or identity of at least 70%, 75%, 80%, 85%, 90%, 95%, 96%, 97%, 98%, 99%, or 99.1 % or higher to the amino acid sequence represented by SEQ ID NO: 1, may consist of an amino acid sequence having the above homology or identity, or may consist essentially of the amino acid sequence. Additionally, it is apparent that any mutant prolyl isomerase having an amino acid sequence, in which part of the sequence is deleted, modified, substituted, conservatively substituted or added, may also fall within the scope of the present disclosure as long as the amino acid sequence has such homology or identity, and exhibits efficacy corresponding to the mutant prolyl isomerase of the present disclosure.

For example, it may be sequence additions or deletions that do not alter the function of the mutant prolyl isomerase of the present disclosure, naturally occurring mutations, silent mutations therein, or conservative substitutions at the N-terminus, C-terminus, and/or within the amino acid sequences.

As used herein, the term "conservative substitution" refers to the substitution of an amino acid with another amino acid having similar structural and/or chemical properties. Such amino acid substitutions may generally occur based on similarity in polarity, charge, solubility, hydrophobicity, hydrophilicity, and/or the amphipathic nature of a residue. Typically, conservative substitutions may have little or no effect on the activity of a protein or polypeptide.

In one example, among electrically charged amino acids, positively charged (basic) amino acids include arginine, lysine, and histidine; negatively charged (acidic) amino acids include glutamic acid and aspartic acid; amino acids having nonpolar side chains (nonpolar amino acids) include glycine, alanine, valine, leucine, isoleucine, methionine, phenylalanine, tryptophan, and proline; amino acids having polar or hydrophilic side chains (polar amino acids) include serine, threonine, cysteine, tyrosine, asparagine, and glutamine. In another example, amino acids having uncharged side chains include glycine, alanine, valine, leucine, isoleucine, methionine, phenylalanine, tryptophan, proline, serine, threonine, cysteine, tyrosine, asparagine, and glutamine. In still another example, aromatic amino acids include phenylalanine, tryptophan, and tyrosine.

As used herein, the term "mutant protein" or "variant" refers to a polypeptide having one or more amino acids different from the amino acid sequence before mutation of the variant by conservative substitutions and/or modifications such that the functions and properties of the polypeptide are retained. Such variants may generally be identified by modifying one or more of the amino acid sequences of the polypeptide and evaluating the properties of the modified polypeptide. That is, the ability of the variants may be enhanced, unchanged or reduced relative to a polypeptide before mutation. Further, some variants may include those in which one or more regions, such as an N-terminal leader sequence or transmembrane domain, have been removed. Moreover, other variants may include those in which a region has been removed from the N- and/or C-terminal of a mature protein. The term "mutant protein" may be used interchangeably with terms such as modification, modified polypeptide, modified protein, mutant, mutein, divergent, *etc.,* without limitation, as long as the terms are used to indicate mutation. In one example, the variant may be a polypeptide in which the amino acid corresponding to position 95 in the amino acid sequence of SEQ ID NO: 1 is methionine, serine, threonine, asparagine, cysteine, histidine, lysine, aspartic acid, alanine, valine, leucine, glutamine, glycine, proline, glutamic acid, arginine, isoleucine, phenylalanine, or tryptophan. In another example, the variant may be a polypeptide in which the amino acid corresponding to position 95 in the amino acid sequence of SEQ ID NO: 1 is substituted with methionine, serine, threonine, asparagine, cysteine, histidine, lysine, aspartic acid, alanine, valine, leucine, glutamine, glycine, proline, or glutamic acid. In still another example, the variant may be a polypeptide including an amino acid sequence represented by SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, or SEQ ID NO: 31, but it is not limited thereto.

Additionally, the variant may also include deletion or addition of amino acids that have minimal influence on the properties and secondary structure of a polypeptide. For example, the polypeptide may be conjugated with a signal (or leader) sequence at the N-terminal involved in the translocation of proteins co-translationally or post-translationally. Further, the variant may also be conjugated with another sequence or linker to identify, purify, or synthesize the polypeptide.

As used herein, the term 'homology' or 'identity' refers to the degree of similarity between two given amino acid sequences or nucleotide sequences, and may be expressed as a percentage. The terms homology and identity may often be used interchangeably with each other.

The sequence homology or identity of conserved polynucleotides or polypeptides may be determined by standard alignment algorithms and can be used with a default gap penalty established by the program being used. Substantially, homologous or identical sequences are generally expected to hybridize to all or part of the sequences under moderate or high stringent conditions. It is apparent that hybridization with polynucleotides containing general codons or degenerate codons in hybridizing polynucleotides is also included.

Whether any two polynucleotide or polypeptide sequences have homology, similarity, or identity may, for example, be determined by a known computer algorithm such as the "FASTA" program (Pearson et al., (1988) [Proc. Natl. Acad. Sci. USA 85]: 2444) using default parameters. Alternatively, it may be determined by the Needleman-Wunsch algorithm (Needleman and Wunsch, 1970, J. Mol. Biol. 48: 443-453), which is performed using the Needleman program of the EMBOSS package (EMBOSS: The European Molecular Biology Open Software Suite, Rice et al., 2000, Trends Genet. 16: 276-277) (preferably, version 5.0.0 or versions thereafter) (GCG program package (Devereux, J., et al., Nucleic Acids Research 12: 387 (1984)), BLASTP, BLASTN, FASTA (Atschul, [S.] [F.,] [ET AL., J MOLEC BIOL 215]: 403 (1990); Guide to Huge Computers, Martin J. Bishop, [ED.,] Academic Press, San Diego, 1994, and [CARILLO ETA/.](1988) SIAM J Applied Math 48: 1073). For example, homology, similarity, or identity may be determined using BLAST or ClustalW of the National Center for Biotechnology Information (NCBI).

The homology, similarity, or identity of polynucleotides or polypeptides may, for example, be determined by comparing sequence information using, for example, the GAP computer program, such as Needleman et al. (1970), J Mol Biol. 48: 443 as disclosed by Smith and Waterman, Adv. Appl. Math (1981) 2:482. In summary, the GAP program defines homology, similarity, or identity as the value obtained by dividing the number of similarly aligned symbols (i.e., nucleotides or amino acids) by the total number of symbols in the shorter of the two sequences. Default parameters for the GAP program may include (1) a unary comparison matrix (containing a value of 1 for identities and 0 for non-identities) and the weighted comparison matrix of Gribskov et al. (1986), Nucl. Acids Res. 14:6745, as disclosed in Schwartz and Dayhoff, eds., Atlas of Protein Sequence and Structure, National Biomedical Research Foundation, pp. 353-358 (1979) (or EDNAFULL substitution matrix (EMBOSS version of NCBI NUC4.4)); (2) a penalty of 3.0 for each gap and an additional penalty of 0.10 for each symbol in each gap (or a gap opening penalty of 10 and a gap extension penalty of 0.5); and (3) no penalty for end gaps.

In one example of the present disclosure, the mutant prolyl isomerase of the present disclosure may have an activity of increasing the production ability of glutamic acid-based amino acids compared to a wild-type polypeptide having prolyl isomerase activity.

As used herein, the term "corresponding to" refers to an amino acid residue at the position recited in a peptide or an amino acid residue that is similar, identical, or homologous to the residue recited in a peptide. Identifying an amino acid at a corresponding position may be determining a particular amino acid in a sequence that refers to a particular sequence. As used herein, the "corresponding region" generally refers to a similar or corresponding position in a related protein or reference protein.

For example, any amino acid sequence is aligned with SEQ ID NO: 1, and based on the alignment, each amino acid residue of the amino acid sequence can be numbered with reference to the numerical position of the amino acid residue corresponding to the amino acid residue of SEQ ID NO: 1. For example, a sequence alignment algorithm such as that described herein can identify the position of an amino acid or a position where modifications such as substitutions, insertions or deletions occur compared to a query sequence (also referred to as a "reference sequence").

Examples of alignment may be determined by the Needleman-Wunsch algorithm (Needleman and Wunsch, 1970, J. Mol. Biol. 48: 443-453), which is performed using the Needleman program of the EMBOSS package (EMBOSS: The European Molecular Biology Open Software Suite, Rice et al., 2000, Trends Genet. 16: 276-277), *etc.,* but is not limited thereto, and sequence alignment programs, such as pairwise sequence comparison algorithms, *etc.,* known in the art may be appropriately used.

Another aspect of the present disclosure provides a polynucleotide encoding the mutant prolyl isomerase of the present disclosure.

The FkpA protein of the present disclosure may be encoded by an *fkpA* gene.

In one example, the *fkpA* gene may be a polynucleotide encoding NCgl0796 (Cg0950) derived from a microorganism of the genus *Corynebacterium,* but it is not limited thereto. In another example, the *fkpA* gene may be an *fkpA* gene derived from a microorganism of the genus *Corynebacterium,* but it is not limited thereto, and it is apparent that the *fkpA* gene includes *fkpA* genes of various origins which encode proteins having FkpA protein activity.

As used herein, the term "polynucleotide", which is a polymer of nucleotides composed of nucleotide monomers connected in a lengthy chain by a covalent bond, is a DNA or RNA strand having at least a certain length. More specifically, it may mean a polynucleotide fragment encoding the mutant prolyl isomerase.

The polynucleotide encoding the mutant prolyl isomerase of the present disclosure may include a nucleotide sequence encoding the mutant prolyl isomerase, in which the amino acid corresponding to position 95 in the amino acid sequence of SEQ ID NO: 1 is substituted with another amino acid, or a nucleotide sequence in which the codons corresponding to positions 283 to 285 in a nucleotide sequence of SEQ ID NO: 2 are substituted with codons encoding other amino acids. In one example, the polynucleotide of the present disclosure may include a nucleotide sequence encoding an amino acid sequence represented by SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, or SEQ ID NO: 31. In a more specific embodiment of the present disclosure, the polynucleotide of the present disclosure may have or include a sequence of SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, or SEQ ID NO: 32. Additionally, the polynucleotide of the present disclosure may consist of or consist essentially of a sequence of SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, or SEQ ID NO: 32.

The polynucleotide of the present disclosure may undergo various modifications in the coding region within the scope that does not change the amino acid sequence of the mutant prolyl isomerase of the present disclosure, due to codon degeneracy or in consideration of the codons preferred in an organism in which the mutant prolyl isomerase of the present disclosure is to be expressed. Specifically, the polynucleotide of the present disclosure may have or include a nucleotide sequence having a homology or identity of 70% or more, 75% or more, 80% or more, 85% or more, 90% or more, 95% or more, 96% or more, 97% or more, 98% or more, and less than 100% to a nucleotide sequence of SEQ ID NO: 2, or may consist of or consist essentially of a nucleotide sequence having a homology or identity of 70% or more, 75% or more, 80% or more, 85% or more, 90% or more, 95% or more, 96% or more, 97% or more, 98% or more, and less than 100% to a nucleotide sequence of SEQ ID NO: 2, but it is not limited thereto. In particular, in the sequence having the above homology or identity, the codon encoding the amino acid corresponding to position 95 of SEQ ID NO: 1 may be one of the codons encoding an amino acid other than tyrosine, for example, methionine, serine, threonine, asparagine, cysteine, histidine, lysine, aspartic acid, alanine, valine, leucine, glutamine, glycine, proline, or glutamic acid.

Additionally, the polynucleotide of the present disclosure may include a probe that may be prepared from a known gene sequence, for example, any sequence which may hybridize with a sequence complementary to all or part of the polynucleotide sequence of the present disclosure under stringent conditions without limitation. The term "stringent conditions" refers to conditions under which specific hybridization between polynucleotides is allowed. Such conditions are specifically described in the literature (e.g., J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York).

For example, the stringent conditions may include conditions under which polynucleotides having a high homology or identity, i.e., polynucleotides having 70% or more, 75% or more, 80% or more, 85% or more, 90% or more, 95% or more, 96% or more, 97% or more, 98% or more, or 99% or more hybridize with each other, while polynucleotides having a homology or identity lower than the above homology or identity do not hybridize with each other; or may include ordinary washing conditions of Southern hybridization, *i.e.,* washing once, specifically twice or three times, at a salt concentration and a temperature corresponding to 60°C., 1×SSC, 0.1% SDS, specifically 60°C., 0.1 ×SSC, 0.1 % SDS, and more specifically 68°C., 0.1 ×SSC, 0.1% SDS.

Hybridization requires that two nucleic acids have complementary sequences, although mismatches between bases are possible depending on the stringency of the hybridization. The term "complementary" is used to describe a relationship between nucleotide bases that may hybridize with each other. For example, with respect to DNA, adenine is complementary to thymine, and cytosine is complementary to guanine. Therefore, the polynucleotide of the present disclosure may also include an isolated nucleic acid fragment complementary to the entire sequence as well as a nucleic sequence substantially similar thereto.

Specifically, polynucleotides having a homology or identity to the polynucleotide of the present disclosure may be detected using the hybridization conditions including a hybridization step at a Tₘ value of 55°C under the above-described conditions. Further, the Tₘ value may be 60°C, 63°C, or 65°C, but is not limited thereto, and may be appropriately adjusted by those skilled in the art depending on the purpose thereof.

The appropriate stringency for hybridizing the polynucleotides depends on the length of the polynucleotides and the degree of complementation, and these variables are well known in the art (*e.g.,* Sambrook et al.).

Still another aspect of the present disclosure provides a vector containing the polynucleotide of the present disclosure.

The vector may be an expression vector for expressing the polynucleotide in a host cell, but it is not limited thereto.

The vector of the present disclosure may include a DNA construct containing the nucleotide sequence of a polynucleotide encoding a target polypeptide operably linked to a suitable expression regulatory region (expression regulatory sequence) so as to be able to express the target polypeptide in a suitable host cell. The expression regulatory sequence may include a promoter capable of initiating transcription, any operator sequence for regulating the transcription, a sequence encoding a suitable mRNA ribosome-binding site, and a sequence for regulating the termination of transcription and translation. Once transformed into a suitable host cell, the vector may replicate or function independently from the host genome, or may integrate into the genome thereof.

The vector used in the present disclosure is not particularly limited, and any vector known in the art may be used. Examples of vectors typically used may include natural or recombinant plasmids, cosmids, viruses, and bacteriophages. For example, as a phage vector or cosmid vector, pWE15, M13, MBL3, MBL4, IXII, ASHII, APII, t10, t11, Charon4A, and Charon21A, *etc*. may be used; and as a plasmid vector, those based on pDZ, pBR, pUC, pBluescriptll, pGEM, pTZ, pCL and pET, *etc.* may be used. Specifically, pDZ, pDC, pDCM2, pACYC177, pACYC184, pCL, pECCG117, pUC19, pBR322, pMW118, pCC1BAC vector, *etc.* may be used.

In one example, a polynucleotide encoding a target polypeptide may be inserted into the chromosome through a vector for intracellular chromosomal insertion. The insertion of the polynucleotide into the chromosome may be performed by any method known in the art, for example, by homologous recombination, but it is not limited thereto. The vector may further include a selection marker to confirm the insertion into the chromosome. The selection marker is for selecting the cells transformed with the vector, that is, for confirming whether the target nucleic acid molecule has been inserted, and markers that provide selectable phenotypes, such as drug resistance, auxotrophy, resistance to cell-toxic agents, or expression of surface polypeptides, may be used. Only cells expressing the selection marker are able to survive or show different phenotypes under the environment treated with the selective agent, and thus the transformed cells may be selected.

As used herein, the term "transformation" refers to the introduction of a vector containing a polynucleotide encoding a target polypeptide into a host cell or a microorganism so that the polypeptide encoded by the polynucleotide can be expressed in the host cell. As long as the transformed polynucleotide can be expressed in the host cell, it does not matter whether the transformed polynucleotide is integrated into the chromosome and located therein, or located extrachromosomally, and both cases can be included. Additionally, the polynucleotide may include DNA and/or RNA encoding the target polypeptide. The polynucleotide may be introduced in any form, as long as it can be introduced into the host cell and expressed therein. For example, the polynucleotide may be introduced into the host cell in the form of an expression cassette, which is a gene construct including all elements required for its autonomous expression. The expression cassette may commonly include a promoter operably linked to the polynucleotide, a transcription terminator, a ribosome-binding site, or a translation terminator. The expression cassette may be in the form of a self-replicable expression vector. Additionally, the polynucleotide may be introduced into a host cell as it is and operably linked to sequences required for expression in the host cell, but it is not limited thereto.

Further, as used herein, the term "operably linked" means that the polynucleotide sequence is functionally linked to a promoter sequence that initiates and mediates transcription of the polynucleotide encoding the target mutant prolyl isomerase of the present disclosure.

Still another aspect of the present disclosure provides a microorganism including the mutant prolyl isomerase of the present disclosure or the polynucleotide of the present disclosure.

The strain of the present disclosure may include the mutant prolyl isomerase of the present disclosure, a polynucleotide encoding the polypeptide, or a vector containing the polynucleotide of the present disclosure.

As used herein, the term "microorganism (or strain)" includes all wild-type microorganisms, or naturally or artificially genetically modified microorganisms, and it may be a microorganism in which a particular mechanism is weakened or enhanced due to insertion of a foreign gene, or enhancement or inactivation of the activity of an endogenous gene, *etc.*, and may be a microorganism including genetic modification to produce a desired polypeptide, protein or product.

The strain of the present disclosure may be a microorganism naturally having a production ability of glutamic acid-based amino acids; or a microorganism in which a production ability of glutamic acid-based amino acids has been introduced into a strain not having a production ability of glutamic acid-based amino acids. In one example, it may be a microorganism in which the mutant prolyl isomerase or a polynucleotide encoding the same has been introduced, and thus has an increased production ability of glutamic acid-based amino acids, but it is not limited thereto.

As used herein, the term "glutamic acid-based amino acid" may include all L-amino acids that can be biosynthesized using glutamic acid as a precursor, including glutamic acid. In one example, L-amino acids that can be produced through the glutamic acid biosynthetic pathway using glutamic acid as a precursor may include glutamine, ornithine, citrulline, and arginine, *etc.,* and in addition, any L-amino acid that can be biosynthesized using glutamic acid as a precursor may be included in the scope of the present disclosure. Additionally, glutamic acid that can be produced by the prolyl isomerase of the present disclosure and any substance synthesized using glutamic acid as a precursor may be included without limitation.

Meanwhile, the term "L-amino acid" used herein includes both proteinogenic and non- proteinogenic amino acids.

The strain of the present disclosure may be a microorganism with an increased production ability of glutamic acid-based amino acids as compared to a parent strain not including the mutant prolyl isomerase of the present disclosure or a wild-type strain of the genus *Corynebacterium.* The microorganism may have an increased production ability of glutamic acid-based amino acids by introducing the mutant prolyl isomerase of the present disclosure.

For example, the non-modified microorganism of prolyl isomerase, which is a control strain for comparing the increase in the production ability of glutamic acid-based amino acids, may be the C. *glutamicum* ATCC 13869 strain, the citrulline-producing C. gl::argR*_argG* strain, the glutamine-producing KFCC10680 strain, the ornithine-producing C. gl::argR*_argF* strain, or the arginine-producing C. gl::argR*strain producing arginine, but it is not limited thereto.

In one example, the recombinant strain with the increased production ability may have an increased production ability of glutamic acid-based amino acids by about 1% or more, specifically about 2% or more, about 5% or more, about 10% or more, about 15% or more, about 20% or more, about 25% or more, about 30% or more, about 31% or more, about 31.9% or more, about 32% or more, about 32.9% or more, about 33% or more, about 33.8% or more, about 34% or more, about 35% or more, about 36% or more, about 36.4% or more, about 37% or more, about 37.2% or more, about 38% or more, about 38.8% or more, about 39% or more, or about 39.5% or more (the upper limit is not particularly limited, for example, about 200% or less, about 150% or less, about 100% or less, or about 50% or less) as compared to the production ability of glutamic acid-based amino acids of a parent strain before modification or a non-modified microorganism, but it is not limited thereto, as long as it has an increased + value compared to the production ability of a parent strain before modification, a non-modified microorganism, or a non-modified microorganism of prolyl isomerase. In another example, the recombinant strain having the increased production ability of glutamic acid-based amino acids may have an increased production ability of glutamic acid-based amino acids by about 1.01 times or more, about 1.02 times or more, about 1.05 times or more, about 1.1 times or more, about 1.15 times or more, about 1.2 times or more, about 1.25 times or more, about 1.3 times or more, about 1.31 times or more, about 1.319 times or more, about 1.32 times or more, about 1.329 times or more, about 1.33 times or more, about 1.338 times or more, about 1.34 times or more, about 1.35 times or more, about 1.36 times or more, about 1.364 times or more, about 1.37 times or more, about 1.372 times or more, about 1.38 times or more, about 1.388 times or more, about 1.39 times or more, or about 1.395 times or more (the upper limit is not particularly limited, for example, about 10 times or less or about 5 times or less), as compared to the production ability of glutamic acid-based amino acids of a parent strain before modification, a non-modified microorganism, or a non-modified microorganism of prolyl isomerase, but it is not limited thereto.

As used herein, the term "non-modified microorganism" does not exclude a strain containing a mutation that may occur naturally in a microorganism, and may refer to a wild-type strain or natural-type strain itself, or a strain before its trait is altered due to genetic modification caused by natural or artificial factors. Additionally, as used herein, the term "non-modified microorganism of prolyl isomerase" may refer to a strain into which the prolyl isomerase variant disclosed herein is not introduced, or a strain before the introduction thereof. The non-modified microorganism of prolyl isomerase of the present disclosure does not exclude strains containing modifications of other proteins or genes in addition to the modifications of the prolyl isomerase or a polynucleotide encoding the same.

As used herein, the term "non-modified microorganism" may be used interchangeably with "strain before modification", "microorganism before modification", "non-mutant strain", "non-modified strain", "non-mutant microorganism", or "reference microorganism".

The microorganism of the present disclosure may be a microorganism including the mutant prolyl isomerase or a polynucleotide encoding the same; or a microorganism (e.g., a recombinant microorganism) that has been genetically modified to include the mutant prolyl isomerase or a polynucleotide encoding the same, but it is not limited thereto. The "endogenous activity" refers to the activity of a particular polypeptide originally possessed by a parent strain before transformation, or a wild-type or non-modified microorganism when a trait is altered by genetic variation due to a natural or artificial factor. The endogenous activity may be interchangeably used with "activity before modification".

In another example of the present disclosure, the microorganism of the present disclosure may be *Corynebacterium glutamicum, Corynebacterium stationis, Corynebacterium crudilactis, Corynebacterium deserti, Corynebacterium efficiens, Corynebacterium callunae, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens,* and specifically, *Corynebacterium glutamicum,* but it is not limited thereto.

In still another example of the present disclosure, the recombinant microorganism of the present disclosure may be a microorganism having an increased production ability of glutamic acid-based amino acids due to enhancement of the activity of part of the proteins involved in the biosynthesis pathway of glutamic acid-based amino acids or weakening of the activity of part of the proteins involved in the degradation pathway of glutamic acid-based amino acids.

In still another example of the present disclosure, the recombinant microorganism of the present disclosure may be a strain with an increased ornithine-producing ability, which is one of the glutamic acid-based amino acids.

In any one of the above-described embodiments, the recombinant microorganism of the present disclosure may be modified to enhance the biosynthesis pathway from glutamate to ornithine, such that one or more activities selected from the group consisting of acetylglutamate synthase, which converts glutamate into N-acetylglutamate; ornithine acetyltransferase (ArgJ) which converts acetylornithine into ornithine; acetylglutamate kinase (ArgB) which converts N-acetylglutamate into N-acetylglutamyl phosphate; acetyl gamma glutamyl phosphate reductase (ArgC) which converts N-acetylglutamyl phosphate into N-acetylglutamate semialdehyde; and acetylornithine aminotransferase (ArgD), which converts N-acetylglutamate semialdehyde into N-acetylornithine, are enhanced as compared to their endogenous activity, thus enhancing ornithine-producing ability.

In still another example of the present disclosure, the recombinant microorganism of the present disclosure may be a strain with an increased citrulline-producing ability, which is one of the glutamic acid-based amino acids.

In any one of the above-described embodiments, the recombinant microorganism of the present disclosure may be modified to enhance the biosynthesis pathway from glutamate to ornithine, such that ornithine carbamoyltransferase (ArgF), which converts ornithine to citrulline, is enhanced as compared to its endogenous activity, and thus enhancing the citrulline-producing ability. Alternatively, the recombinant microorganism of the present disclosure may be further modified to enhance the biosynthesis pathway from glutamate to ornithine, as described above, and thus enhancing ornithine-producing ability, a precursor of citrulline, thereby enhancing citrulline-producing ability.

In still another example of the present disclosure, the recombinant microorganism of the present disclosure may be a strain with an increased arginine-producing ability, which is one of the glutamic acid-based amino acids.

In any one of the above-described embodiments, the recombinant microorganism of the present disclosure may be modified to enhance the biosynthesis pathway from ornithine to arginine, such that one or more activities selected from the group consisting of argininosuccinate synthase (argG), argininosuccinate lyase (argH), aspartate ammonia lyase (aspA), and aspartate aminotransferase (aspB) involved in arginine synthesis are enhanced as compared to its endogenous activity, and thus enhancing the arginine-producing ability. Alternatively, the recombinant microorganism of the present disclosure may be further modified to enhance the biosynthesis pathway from glutamic acid to ornithine, as described above, and thus enhancing ornithine-producing ability, a precursor of citrulline, thereby enhancing arginine-producing ability.

In still another example of the present disclosure, the recombinant microorganism of the present disclosure may be a strain with an increased glutamine-producing ability, which is one of the glutamic acid-based amino acids.

In any one of the above-described embodiments, the recombinant microorganism of the present disclosure may be modified to enhance the biosynthesis pathway from glutamic acid to glutamine, such that glutamine synthetase involved in glutamine synthesis is enhanced as compared to its endogenous activity, and thus enhancing glutamine-producing ability. Alternatively, the recombinant microorganism of the present disclosure may be further modified to enhance the biosynthesis pathway from glutamic acid to glutamine, as described above, and thus enhancing glutamic acid-producing ability, a precursor of glutamine, thereby enhancing glutamine-producing ability.

As used herein, the term "weakening" of the activity of a polypeptide (*e.g.,* proteins specified by the name of each enzyme) is a comprehensive concept including both reduced or no activity compared to its endogenous activity. The weakening may be used interchangeably with terms such as inactivation, deficiency, down-regulation, decrease, reduction, attenuation, *etc.*

The weakening may also include a case where the activity of a polypeptide itself is decreased or removed compared to the activity of the polypeptide originally possessed by a microorganism due to mutation of a polynucleotide encoding the polypeptide, *etc.;* a case where the overall level of intracellular polypeptide activity and/or concentration (expression level) is decreased compared to a natural strain due to the inhibition of expression of a gene of a polynucleotide encoding the polypeptide, or the inhibition of translation into the polypeptide, *etc.;* a case where the polynucleotide is not expressed at all; and/or a case where no polypeptide activity is observed even when the polynucleotide is expressed. The expression that the activity of a polypeptide is "inactivated, deficient, decreased, down-regulated, reduced or attenuated" compared to its endogenous activity means that the activity of a polypeptide is decreased compared to the activity of a particular polypeptide originally possessed by a parent strain before transformation or a non-modified microorganism.

The weakening of the activity of a polypeptide may be performed by any method known in the art, but the method is not limited thereto, and may be achieved by applying various methods well known in the art (*e.g.,* Nakashima N et al., Bacterial cellular engineering by genome editing and gene silencing. Int J Mol Sci. 2014;15(2):2773-2793, Sambrook et al. Molecular Cloning 2012, *etc*.).

Specifically, the weakening of the activity of a polypeptide of the present disclosure may be achieved by:
1) deleting a part or all of a gene encoding the polypeptide;
2) modifying the expression regulatory region (expression regulatory sequence) such that the expression of a gene encoding the polypeptide is decreased;
3) modifying the amino acid sequence constituting the polypeptide such that the polypeptide activity is removed or weakened (*e.g.,* deletion/substitution/addition of one or more amino acids on the amino acid sequence);
4) modifying the gene sequence encoding the polypeptide such that the polypeptide activity is removed or weakened (*e.g.,* deletion/substitution/addition of one or more of nucleotides on the nucleotide sequence of the polypeptide gene to encode a polypeptide that has been modified to remove or weaken the activity of the polypeptide);
5) modifying the nucleotide sequence encoding the initiation codon or 5'-UTR of a gene transcript encoding the polypeptide;
6) introducing an antisense oligonucleotide (*e.g.,* antisense RNA), which binds complementary to a gene transcript encoding the polypeptide;
7) adding a sequence complementary to the Shine-Dalgarno (SD) sequence on the front end of the SD sequence of a gene encoding the polypeptide to form a secondary structure, thereby inhibiting the ribosomal attachment;
8) a reverse transcription engineering (RTE), which adds a promoter, which is to be reversely transcribed, on the 3' terminus of the open reading frame (ORF) of a gene sequence encoding the polypeptide;
9) regulating the cellular localization of the protein (polypeptide); or
10) a combination of two or more selected from above 1 to 9), but is not particularly limited thereto.

For example,
The 1) method of deleting a part or all of a gene encoding the polypeptide may be achieved by deleting all of the polynucleotide encoding the endogenous target polypeptide within the chromosome, or by replacing the polynucleotide with a polynucleotide having a partially deleted nucleotide, or with a marker gene.

The 2) method of modifying the expression regulatory region (expression regulatory sequence) may be achieved by inducing a modification on the expression regulatory region (expression regulatory sequence) through deletion, insertion, non-conservative substitution or conservative substitution, or a combination thereof; or by replacing the sequence with a sequence having a weaker activity. The expression regulatory region may include a promoter, an operator sequence, a sequence encoding a ribosome-binding site, and a sequence for regulating the termination of transcription and translation, but is not limited thereto.

The 3) and 4) methods of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to weaken the activity of the polypeptide, or by replacing the sequence with an amino acid sequence or a polynucleotide sequence modified to have a weaker activity, or an amino acid sequence or a polynucleotide sequence modified to have no activity, but are not limited thereto. For example, the expression of the gene may be inhibited or weakened by introducing a mutation into the polynucleotide sequence to form a termination codon, but is not limited thereto.

The 5) method of modifying the nucleotide sequence encoding the initiation codon or 5'-UTR of a gene transcript encoding the polypeptide may be achieved, for example, by substituting the nucleotide sequence with a nucleotide sequence encoding another initiation codon having a lower polypeptide expression rate than the endogenous initiation codon, but is not limited thereto.

The 6) method of introducing an antisense oligonucleotide (*e.g.,* antisense RNA), which binds complementary to a gene transcript encoding the polypeptide, can be found in the literature [Weintraub, H. et al., Antisense-RNA as a molecular tool for genetic analysis, Reviews - Trends in Genetics, Vol. 1(1) 1986].

The 7) method of adding a sequence complementary to the Shine-Dalgarno (SD) sequence on the front end of the SD sequence of a gene encoding the polypeptide to form a secondary structure, thereby inhibiting the ribosome attachment may be achieved by inhibiting mRNA translation or reducing the speed thereof.

The 8) reverse transcription engineering (RTE), which adds a promoter, which is to be reversely transcribed, on the 3' terminus of the open reading frame (ORF) of a gene sequence encoding the polypeptide, may be achieved by forming an antisense nucleotide complementary to the gene transcript encoding the polypeptide to weaken the activity.

Further, the 9) method of regulating the cellular localization of the protein (polypeptide) may be achieved by targeting the protein (polypeptide) to a particular organelle or a particular space within the cell. For example, it may be achieved by targeting the protein (polypeptide) to the periplasm or cytoplasm through addition or removal of a leader sequence that functions in targeting protein (polypeptide), but is not limited thereto.

Such weakening of the activity of the polypeptide may mean that the activity or concentration of the corresponding polypeptide is weakened relative to the activity or concentration of a polypeptide expressed in a wild-type strain or a microbial strain before modification, or that the amount of products produced from the corresponding polypeptide is decreased, but it is not limited thereto.

As used herein, the term "enhancement" of the activity of a polypeptide means that the activity of a polypeptide is increased compared to its endogenous activity. The enhancement may be used interchangeably with terms such as activation, up-regulation, overexpression, increase, *etc.* In particular, the activation, enhancement, up-regulation, overexpression and increase may include both cases in which an activity not originally possessed is exhibited, or the activity is enhanced compared to the endogenous activity or the activity before modification. The "enhancement", "up-regulation", "overexpression" or "increase" in the activity of a polypeptide compared to its endogenous activity means that the activity and/or concentration (expression level) of the polypeptide is enhanced compared to that of a particular polypeptide originally possessed by a parent strain before transformation or a non-modified microorganism.

The enhancement may be achieved by introducing a foreign polypeptide, or by enhancing the activity and/or concentration (expression level) of the endogenous polypeptide. The enhancement of the activity of the polypeptide may be confirmed by the increase in the level of activity of the polypeptide, expression level, or the amount of product excreted from the polypeptide.

The enhancement of the activity of the polypeptide may be applied by various methods well known in the art, and the method is not limited as long as it can enhance the activity of the target polypeptide compared to that of a microorganism before modification. Specifically, genetic engineering and/or protein engineering well known to those skilled in the art, which are common methods of molecular biology, may be used, but the method is not limited thereto (*e.g.,* Sitnicka et al. Functional Analysis of Genes. Advances in Cell Biology. 2010, Vol. 2. 1-16, Sambrook et al. Molecular Cloning 2012, *etc*.).

Specifically, the enhancement of the activity of the polypeptide of the present disclosure may be achieved by:
1) increasing the intracellular copy number of a polynucleotide encoding the polypeptide;
2) replacing the expression regulatory region of a gene encoding the polypeptide on the chromosome with a sequence having a stronger activity
3) modifying the nucleotide sequence encoding the initiation codon or 5'-UTR of a gene transcript encoding the polypeptide;
4) modifying the amino acid sequence of the polypeptide such that the activity of the polypeptide is enhanced;
5) modifying the polynucleotide sequence encoding the polypeptide such that the activity of the polypeptide is enhanced (*e.g.,* modifying the polynucleotide sequence of the polypeptide gene to encode a polypeptide that has been modified to enhance the activity of the polypeptide);
6) introducing a foreign polypeptide exhibiting the polypeptide activity or a foreign polynucleotide encoding the same;
7) codon-optimization of the polynucleotide encoding the polypeptide;
8) analyzing the tertiary structure of the polypeptide and thereby selecting and modifying the exposed site, or chemically modifying the same;
9) regulating the cellular localization of the protein (polypeptide); or
10) a combination of two or more selected from above 1 to 9), but is not particularly limited thereto.

More specifically,
The 1) method of increasing the intracellular copy number of a polynucleotide encoding the polypeptide may be achieved by introducing a vector, which is operably linked to the polynucleotide encoding the polypeptide and is able to replicate and function regardless of a host cell, into the host cell. Alternatively, the method may be achieved by introducing one copy or two copies of polynucleotides encoding the polypeptide into the chromosome of a host cell. The introduction into the chromosome may be performed by introducing a vector, which is able to insert the polynucleotide into the chromosome of a host cell, into the host cell, but is not limited thereto. The vector is as described above.

The 2) method of replacing the expression regulatory region (or expression regulatory sequence) of a gene encoding the polypeptide on the chromosome with a sequence having a strong activity may be achieved, for example, by inducing a modification on the sequence through deletion, insertion, non-conservative or conservative substitution, or a combination thereof to further enhance the activity of the expression regulatory region, or by replacing the sequence with a sequence having a stronger activity. The expression regulatory region may include, but is not particularly limited to, a promoter, an operator sequence, a sequence encoding a ribosome-binding site, and a sequence regulating the termination of transcription and translation, *etc.* In one example, the method may include replacing the original promoter with a strong promoter, but is not limited thereto.

Examples of the known strong promoter may include CJ1 to CJ7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, *etc.,* but the strong promoter is not limited thereto.

The 3) method of modifying the nucleotide sequence encoding the initiation codon or 5'-UTR of a gene transcript encoding the polypeptide may be achieved, for example, by substituting the nucleotide sequence with a nucleotide sequence encoding another initiation codon having a higher expression rate of the polypeptide compared to the endogenous initiation codon, but is not limited thereto.

The 4) and 5) methods of modifying the amino acid sequence or the polynucleotide sequence may be achieved by inducing a modification on the sequence through deletion, insertion, non-conservative or conservative substitution of the amino acid sequence of the polypeptide or the polynucleotide sequence encoding the polypeptide, or a combination thereof to enhance the activity of the polypeptide, or by replacing the sequence with an amino acid sequence or a polynucleotide sequence modified to have a stronger activity, or an amino acid sequence or a polynucleotide sequence modified to enhance the activity, but are not limited thereto. The replacement may specifically be performed by inserting the polynucleotide into the chromosome by homologous recombination, but is not limited thereto. The vector used herein may further include a selection marker to confirm the insertion into the chromosome.

The 6) method of introducing a foreign polynucleotide exhibiting the activity of the polypeptide may be achieved by introducing into a host cell a foreign polynucleotide encoding a polypeptide that exhibits the same/similar activity to that of the polypeptide. The foreign polynucleotide may be used without limitation regardless of its origin or sequence as long as it exhibits the same/similar activity to that of the polypeptide. The introduction may be performed by those of ordinary skill in the art by appropriately selecting a transformation method known in the art, and the expression of the introduced polynucleotide in the host cell enables to produce the polypeptide, thereby increasing its activity.

The 7) method of codon-optimization of the polynucleotide encoding the polypeptide may be achieved by codon-optimization of an endogenous polynucleotide to increase the transcription or translation within a host cell, or by optimizing the codons thereof such that the optimized transcription and translation of the foreign polynucleotide can be achieved within the host cell.

The 8) method of analyzing the tertiary structure of the polypeptide and thereby selecting and modifying the exposed site, or chemically modifying the same may be achieved, for example, by comparing the sequence information of the polypeptide to be analyzed with a database, in which the sequence information of known proteins is stored, to determine template protein candidates according to the degree of sequence similarity, and thus confirming the structure based on the information, thereby selecting and transforming or modifying the exposed site to be modified or chemically modified.

The 9) method of regulating the cellular localization of the protein (polypeptide) may be achieved by targeting the protein (polypeptide) to a particular organelle or a particular space within the cell. For example, it may be achieved by targeting the protein (polypeptide) to the periplasm or cytoplasm through addition or removal of a leader sequence that functions in targeting protein (polypeptide), but is not limited thereto.

Such enhancement of the activity of the polypeptide may mean that the activity or concentration of the corresponding polypeptide is increased relative to the activity or concentration of the polypeptide expressed in a wild-type strain or a microbial strain before modification, or that the amount of product produced from the polypeptide is increased, but is not limited thereto.

The modification of a part or all of the polynucleotide in the microorganism of the present disclosure (a) may be achieved by homologous recombination using a vector for chromosomal insertion in the microorganism or genome editing using an engineered nuclease (*e.g.,* CRISPR-Cas9), and/or (b) may be induced by light, such as ultraviolet rays and radiation, *etc.* and/or chemical treatments, but is not limited thereto. The method of modifying a part or all of the gene may include a method using DNA recombination technology. For example, a part or all of the gene may be deleted by injecting a nucleotide sequence or a vector containing a nucleotide sequence homologous to the target gene into the microorganism to induce homologous recombination. The injected nucleotide sequence or the vector may include a dominant selection marker, but it is not limited thereto.

In the microorganism of the present disclosure, the mutant prolyl isomerase, polynucleotide, and glutamic acid-based amino acids, *etc.* are as described in other aspects above.

Still another aspect of the present disclosure provides a method for producing glutamic acid-based amino acids, including: culturing a microorganism, which includes the mutant prolyl isomerase of the present disclosure or the polypeptide of the present disclosure, in a medium.

The method for producing glutamic acid-based amino acids of the present disclosure may include a step of culturing a microorganism, which includes the mutant prolyl isomerase of the present disclosure, the polypeptide of the present disclosure, or the vector of the present disclosure, in a medium.

As used herein, the term "cultivation" means that the microorganism of the present disclosure is grown under appropriately controlled environmental conditions. The cultivation process of the present disclosure may be performed in a suitable culture medium and culture conditions known in the art. Such a cultivation process may be easily adjusted for use by those skilled in the art according to the microorganism to be selected. Specifically, the cultivation may be a batch culture, a continuous culture, and/or a fed-batch culture, but it is not limited thereto.

As used herein, the term "medium" refers to a mixture of materials which contains nutrient materials required for the cultivation of the microorganism of the present disclosure as a main ingredient, and it supplies nutrient materials and growth factors, along with water that is essential for survival and growth. Specifically, the medium and other culture conditions used for culturing the microorganism of the present disclosure may be any medium used for conventional cultivation of microorganisms without any particular limitation. However, the microorganism of the present disclosure may be cultured under aerobic conditions in a conventional medium containing an appropriate carbon source, nitrogen source, phosphorus source, inorganic compound, amino acid, and/or vitamin, *etc.,* while adjusting temperature, pH, *etc.*

Specifically, the culture medium for the microorganism of the genus *Corynebacterium* can be found in the literature ["Manual of Methods for General Bacteriology" by the American Society for Bacteriology (Washington D.C., USA, 1981)].

In the present disclosure, the carbon source may include carbohydrates, such as glucose, saccharose, lactose, fructose, sucrose, maltose, *etc.;* sugar alcohols, such as mannitol, sorbitol, *etc.;* organic acids, such as pyruvic acid, lactic acid, citric acid, *etc.;* and amino acids, such as glutamic acid, methionine, lysine, *etc.* Additionally, the carbon source may include natural organic nutrients such as starch hydrolysate, molasses, blackstrap molasses, rice bran, cassava, sugar cane molasses, and corn steep liquor, *etc.* Specifically, carbohydrates such as glucose and sterilized pretreated molasses (*i.e*., molasses converted to reducing sugar) may be used, and in addition, various other carbon sources in an appropriate amount may be used without limitation. These carbon sources may be used alone or in a combination of two or more kinds, but they are not limited thereto.

The nitrogen source may include inorganic nitrogen sources, such as ammonia, ammonium sulfate, ammonium chloride, ammonium acetate, ammonium phosphate, ammonium carbonate, ammonium nitrate, *etc.;* amino acids, such as glutamic acid, methionine, glutamine, *etc.;* and organic nitrogen sources, such as peptone, NZ-amine, meat extract, yeast extract, malt extract, corn steep liquor, casein hydrolysate, fish or decomposition products thereof, defatted soybean cake or decomposition products thereof, *etc.* These nitrogen sources may be used alone or in a combination of two or more kinds, but they are not limited thereto.

The phosphorus source may include monopotassium phosphate, dipotassium phosphate, or corresponding sodium-containing salts, *etc.* Examples of the inorganic compounds may include sodium chloride, calcium chloride, iron chloride, magnesium sulfate, iron sulfate, manganese sulfate, calcium carbonate, *etc.* Additionally, amino acids, vitamins, and/or appropriate precursors, *etc.,* may be included. These constituting ingredients or precursors may be added to a medium in a batch or continuous manner, but these phosphorus sources are not limited thereto.

Additionally, the pH of the medium may be adjusted by adding a compound such as ammonium hydroxide, potassium hydroxide, ammonia, phosphoric acid, sulfuric acid, *etc.,* during the cultivation of the microorganism of the present disclosure in an appropriate manner. In addition, bubble formation may be prevented during the cultivation using an antifoaming agent such as fatty acid polyglycol ester. Further, oxygen gas or a gas containing oxygen may be injected into the medium in order to maintain aerobic conditions of the medium; or nitrogen gas, hydrogen gas, or carbon dioxide may be injected to maintain anaerobic or microaerobic conditions, without the injection of gas, but the gas is not limited thereto.

The temperature during the cultivation of the present disclosure may be in the range from 20°C to 45°C, specifically from 25°C to 40°C, and the cultivation may be continued for about 10 hours to 160 hours, but it is not limited thereto.

The glutamic acid-based amino acids produced by the cultivation of the present disclosure may be released into the medium or remain in the cells. Herein, the glutamic acid-based amino acids are as described above.

The method for producing glutamic acid-based amino acids of the present disclosure may further include a step of preparing the microorganism of the present disclosure, a step of preparing a medium for culturing the microorganism, or a combination thereof (regardless of the order, in any order), for example, prior to the culturing step.

The method for producing glutamic acid-based amino acids of the present disclosure may further include a step of recovering the glutamic acid-based amino acids from the cultured medium (medium on which the cultivation was performed), or from a strain of *Corynebacterium glutamicum.* The recovering step may be further included after the culturing step.

In the recovering step, the desired glutamic acid-based amino acids may be collected using the method of culturing the microorganism of the present disclosure, for example, using a suitable method known in the art according to a batch culture, continuous culture, or fed-batch culture method, *etc.* For example, methods such as centrifugation, filtration, treatment with a protein-crystallizing precipitant (salting-out method), extraction, ultrasonic disruption, ultrafiltration, dialysis, various kinds of chromatographies such as molecular sieve chromatography (gel filtration), adsorption chromatography, ion exchange chromatography, affinity chromatography, *etc.,* HPLC or a combination thereof may be used, and the desired glutamic acid-based amino acids can be recovered from the medium or microorganism using suitable methods known in the art.

Additionally, the method for producing glutamic acid-based amino acids of the present disclosure may further include a purification step, which may be performed using an appropriate method known in the art. In one example, when the method for producing glutamic acid-based amino acids of the present disclosure includes both a recovering step and a purification step, the recovering step and the purification step may be performed continuously or intermittently regardless of the order, or simultaneously, or may be integrated into one step, but the method is not limited thereto.

In the method of the present disclosure, the mutant prolyl isomerase, polynucleotide, vector, strain, and glutamic acid-based amino acids, *etc.* are as described in other aspects above.

Still another aspect of the present disclosure provides a composition for producing glutamic acid-based amino acids, including: the mutant prolyl isomerase of the present disclosure, a polypeptide encoding the same, a vector containing the polynucleotide, or a microorganism including the polynucleotide; a medium on which the microorganism is cultured; or two or more thereof.

The composition of the present disclosure may further include any suitable excipient commonly used in compositions for producing L-amino acids, and such excipients may include, for example, preservatives, wetting agents, dispersing agents, suspending agents, buffers, stabilizers, or isotonic agents, *etc.,* but are not limited thereto.

Still another aspect of the present disclosure provides use of the mutant prolyl isomerase of the present disclosure for production of glutamic acid-based amino acids.

Still another aspect of the present disclosure provides use of the mutant prolyl isomerase of the present disclosure, a polypeptide encoding the same, a vector containing the polynucleotide, or a microorganism including the polynucleotide for production of glutamic acid-based amino acids

The prolyl isomerase, mutant prolyl isomerase, polynucleotide, vector, strain, medium, and glutamic acid-based amino acids, *etc.* are as described in other aspects above.

### [Mode for Carrying Out the Invention]

Hereinafter, the present disclosure will be described in detail by way of Examples. However, these Examples are merely preferred Examples given for illustrative purposes, and thus, the scope of the invention is not intended to be limited to or by these Examples. Meanwhile, technical features that are not described herein can be sufficiently understood and easily carried out by those skilled in the art in the technical field of the present disclosure or in a similar technical field.

### Example 1: Preparation of Mutant Prolyl Isomerase Expression Vector

Vectors were prepared to introduce Y95M, Y95S, Y95T, Y95N, Y95C, Y95H, Y95K, Y95D, Y95A, Y95V, Y95L, Y95Q, Y95G, Y95P, and Y95E point mutations into the *fkpA* gene (SEQ ID NO: 2) encoding prolyl isomerase (FkpA) in a wild-type *Corynebacterium glutamicum.*

In order to prepare vectors for introducing each mutation, the genome of the wild-type *Corynebacterium glutamicum* ATCC 13869 was used as a template to amplify the homologous recombinant A arm and homologous recombinant B arm, and the primer pairs used for each substituted residue are as shown in Table 1 below.

**[Table 1]**

| Substituted Residue | Primer pairs used to amplify homologous recombinant A arm | Primer pairs used to amplify homologous recombinant B arm |
|---|---|---|
| Methionine (Met) | SEQ ID NOS: 33 and 34 | SEQ ID NOS: 35 and 50 |
| Serine (Ser) | SEQ ID NOS: 33 and 34 | SEQ ID NOS: 36 and 50 |
| Threonine (Thr) | SEQ ID NOS: 33 and 34 | SEQ ID NOS: 37 and 50 |
| Asparagine (Asn) | SEQ ID NOS: 33 and 34 | SEQ ID NOS: 38 and 50 |
| Cysteine (Cys) | SEQ ID NOS: 33 and 34 | SEQ ID NOS: 39 and 50 |
| Histidine (His) | SEQ ID NOS: 33 and 34 | SEQ ID NOS: 40 and 50 |
| Lysine (Lys) | SEQ ID NOS: 33 and 34 | SEQ ID NOS: 41 and 50 |
| Aspartic acid (Asp) | SEQ ID NOS: 33 and 34 | SEQ ID NOS: 42 and 50 |
| Alanine (Ala) | SEQ ID NOS: 33 and 34 | SEQ ID NOS: 43 and 50 |
| Valine (Val) | SEQ ID NOS: 33 and 34 | SEQ ID NOS: 44 and 50 |
| Leucine (Leu) | SEQ ID NOS: 33 and 34 | SEQ ID NOS: 45 and 50 |
| Glutamine (Gln) | SEQ ID NOS: 33 and 34 | SEQ ID NOS: 46 and 50 |
| Glycine (Gly) | SEQ ID NOS: 33 and 34 | SEQ ID NOS: 47 and 50 |
| Proline (Pro) | SEQ ID NOS: 33 and 34 | SEQ ID NOS: 48 and 50 |
| Glutamic acid (Glu) | SEQ ID NOS: 33 and 34 | SEQ ID NOS: 49 and 50 |

PCR was performed under the conditions of denaturation at 95°C for 5 minutes, followed by 30 cycles of denaturation at 95°C for 30 seconds, annealing at 55°C for 30 seconds, and polymerization at 72°C for 1 minute, and then polymerization at 72°C for 5 minutes. Thereafter, PCR fragments were extracted using the gel purification kit (QIAGEN). The thus-obtained gene fragments of homologous recombinant A arm and B arm, and the pDCM2 vector (US 2023-0134555 A1) cleaved by BamHI and Xbal restriction enzymes were linked using the Gibson assembly method (DG Gibson et al., NATURE METHODS, VOL.6 NO.5, MAY 2009, NEBuilder HiFi DNA Assembly Master Mix). The vectors were each transformed into *E*. *coli* DH5α, which was then plated on LB solid medium containing kanamycin (25 mg/l). PCR was performed using a primer pair of SEQ ID NOS: 51 and 52 to select the transformed colonies, and plasmids were obtained using a conventional plasmid extraction method known in the art, which were each named pDCM2-fkpA(Y95M), pDCM2-fkpA(Y95S), pDCM2-fkpA(Y95T), pDCM2-fkpA(Y95N), pDCM2-fkpA(Y95C), pDCM2-fkpA(Y95H), pDCM2-fkpA(Y95K), pDCM2-fkpA(Y95D), pDCM2-fkpA(Y95A), pDCM2-fkpA(Y95V), pDCM2-fkpA(Y95L), pDCM2-fkpA(Y95Q), pDCM2-fkpA(Y95G), pDCM2-fkpA(Y95P), and pDCM2-fkpA(Y95E)

The primer sequences used herein are shown in Table 2 below.

**[Table 2]**

| SEQ ID NO: | Sequence Name | Sequences (5' -> 3') |
|---|---|---|
| 33 | primer 1 | TCGGTACCCGGGGATCCGTATTGTTCGCAATCG |
| 34 | primer 2 | AGCAGCCTCTGGCGGAATGGTCAGC |
| 35 | primer 3 | TCCGCCAGAGGCTGCTATGGGCCCTGAGGGTTCCG |
| 36 | primer 4 | TCCGCCAGAGGCTGCTTCCGGCCCTGAGGGTTCCG |
| 37 | primer 5 | TCCGCCAGAGGCTGCTACCGGCCCTGAGGGTTCCG |
| 38 | primer 6 | TCCGCCAGAGGCTGCTAACGGCCCTGAGGGTTCCG |
| 39 | primer 7 | TCCGCCAGAGGCTGCTTGCGGCCCTGAGGGTTCCG |
| 40 | primer 8 | TCCGCCAGAGGCTGCTCACGGCCCTGAGGGTTCCG |
| 41 | primer 9 | TCCGCCAGAGGCTGCTAAGGGCCCTGAGGGTTCCG |
| 42 | primer 10 | TCCGCCAGAGGCTGCTGATGGCCCTGAGGGTTCCG |
| 43 | primer 11 | TCCGCCAGAGGCTGCTGCAGGCCCTGAGGGTTCCG |
| 44 | primer 12 | TCCGCCAGAGGCTGCTGTGGGCCCTGAGGGTTCCG |
| 45 | primer 13 | TCCGCCAGAGGCTGCTCTGGGCCCTGAGGGTTCCG |
| 46 | primer 14 | TCCGCCAGAGGCTGCTCAGGGCCCTGAGGGTTCCG |
| 47 | primer 15 | TCCGCCAGAGGCTGCTGGCGGCCCTGAGGGTTCCG |
| 48 | primer 16 | TCCGCCAGAGGCTGCTCCAGGCCCTGAGGGTTCCG |
| 49 | primer 17 | TCCGCCAGAGGCTGCTGAAGGCCCTGAGGGTTCCG |
| 50 | primer 18 | TGCAGGTCGACTCTAGACTGCCGATCGTGTCCATC |
| 51 | primer 19 | TATTACGCCAGCTGGCGAAA |
| 52 | primer 20 | GCTTTACACTTTATGCTTCC |

### Example 2: Evaluation of L-Citrulline-Producing Ability

### Example 2-1. Preparation of Control Group

In order to prepare a control strain for the evaluation experiment of L-citrulline-producing ability, a vector was constructed, in which the vector replaces glutamic acid at position 47 of a protein sequence of argR(ANU33619.1) with a stop codon. The genome of wild-type C. *glutamicum* ATCC 13869 was used as a template, and homologous recombinant A arm and homologous recombinant B arm were amplified using a primer pair of SEQ ID NOS: 53 and 54 and a primer pair of SEQ ID NOS: 55 and 56, respectively. Thereafter, a plasmid was obtained in the same manner as in Example 1, which was named pDCM2-argR(E47*).

Further, in order to prepare a strain with further enhanced L-citrulline-producing ability, a vector was constructed, in which the vector replaces phenylalanine at position 68 of a protein sequence of argG(ANU33620.1) with a stop codon. The genome of wild-type C. *glutamicum* ATCC 13869 was used as a template, and homologous recombinant A arm and homologous recombinant B arm were amplified using a primer pair of SEQ ID NOS: 57 and 58 and a primer pair of SEQ ID NOS: 59 and 60, respectively. Thereafter, a plasmid was obtained in the same manner as in Example 1, which was named pDCM2- argG(F68*).

The primer sequences used herein are shown in Table 3 below.

**[Table 3]**

| SEQ ID NO: | Sequence Name | Sequences (5' -> 3') |
|---|---|---|
| 53 | primer 21 | CGGTACCCGGGGATCCCTCGTGCGGAATTCGTGGAG |
| 54 | primer 22 | ATCCAGCAGCAATTCAGACA |
| 55 | primer 23 | CTGAATTGCTGCTGGATTAAGGCATCGATATCACCCA |
| 56 | primer 24 | ATGCCTGCAGGTCGACCCTTCATTTTAAGTTCCTTG |
| 57 | primer 25 | CGGTACCCGGGGATCCTTCATCGATAGGGTGGG |
| 58 | primer 26 | GTACTCCTCAGCTTACTCATCCTTTGCATCAACA |
| 59 | primer 27 | AGTAAGCTGAGGAGTACTGCCTGCCAACCATCAA |
| 60 | primer 28 | ATGCCTGCAGGTCGACCGACTGGCTTGCCACCCT |

The wild-type *C. glutamicum* ATCC 13869 was transformed with the pDCM2-argR(E47*) vector prepared above by electroporation (Appl. Microbiol.Biotechnol. (1999) 52:541-545), and then a second crossover process was performed to obtain a strain, in which a nucleotide sequence at position 139 of argR was substituted from guanine (g) to thymine (t) and thus a protein sequence at position 47 was substituted with a stop codon. PCR and sequencing analysis were performed using a primer pair of SEQ ID NOS: 53 and 56 which are capable of amplifying the adjacent region including the position where the corresponding gene was inserted, and the corresponding genetic manipulation was confirmed. The strain thus obtained was named C. gl::argR*.

In order to prepare a strain with further enhanced L-citrulline production in the C. gl::argR* obtained above, the pDCM2-argG(F68*) vector was used to obtain a strain in the same manner as above. PCR and sequencing analysis were performed using a primer pair of SEQ ID NOS: 57 and 60 which are capable of amplifying the adjacent region including the position where the corresponding gene was inserted, and the corresponding genetic manipulation was confirmed. The strain thus obtained was named C. gl::argR*_argG*.

### Example 2-2. Preparation of Mutant Prolyl Isomerase Expression Vector

In order to prepare mutant strains, in which a point mutation was introduced into prolyl isomerase based on the C. gl::argR*_argG* strain obtained in Example 2-1, the C. gl::argR*_argG* strain was transformed with the pDCM2-fkpA(Y95M), pDCM2-fkpA(Y95S), pDCM2-fkpA(Y95T), pDCM2-fkpA(Y95N), pDCM2-fkpA(Y95C), pDCM2-fkpA(Y95H), pDCM2-fkpA(Y95K), pDCM2-fkpA(Y95D), pDCM2-fkpA(Y95A), pDCM2-fkpA(Y95V), pDCM2-fkpA(Y95L), pDCM2-fkpA(Y95Q), pDCM2-fkpA(Y95G), pDCM2-fkpA(Y95P), and pDCM2-fkpA(Y95E) of Example 1 by electroporation, and then a second crossover process was performed to obtain strains, in which the amino acid at position 95 from the N-terminus of prolyl isomerase was substituted from tyrosine to methionine, serine, threonine, asparagine, cysteine, histidine, lysine, aspartic acid, alanine, valine, leucine, glutamine, glycine, proline or glutamic acid, respectively. The DNA fragments including the *fkpA* gene within the chromosome were amplified by PCR using a primer pair of SEQ ID NOS: 33 and 50 in the genome of the thus-obtained strains. PCR was performed under conditions of denaturation at 95°C for 10 minutes, followed by 30 cycles of denaturation at 95°C for 30 seconds, annealing at 55°C for 30 seconds, and polymerization at 72°C for 2 minutes, and then polymerization at 72°C for 10 minutes. The nucleotide sequences of the amplified gene were analyzed, and as a result, it was confirmed that a mutation was introduced into the nucleotide sequences at positions 283 to 285 downstream from the ORF initiation codon of the *fkpA* gene in each strain (Table 4).

**[Table 4]**

| Amino acids before substitution (nucleotide sequence) | Amino acids after substitution (nucleotide sequence) |
|---|---|
| Tyr(TAC) | Met(ATG) |
| | Ser(TCC) |
| | Thr(ACC) |
| | Asn(AAC) |
| | Cys(TGC) |
| | His(CAC) |
| | Lys(AAG) |
| | Asp(GAT) |
| | Ala(GCA) |
| | Val(GTG) |
| | Leu(CTG) |
| | Gln(CAG) |
| | Gly(GGC) |
| | Pro(CCA) |
| | Glu(GAA) |

Accordingly, mutant strains, in which the amino acid at position 95 from the N-terminus of prolyl isomerase was substituted from tyrosine to methionine, serine, threonine, asparagine, cysteine, histidine, lysine, aspartic acid, alanine, valine, leucine, glutamine, glycine, proline or glutamic acid, were obtained based on the C. gl::argR*_argG* strain, respectively, and the thus-obtained mutant strains were named C. gl::argR*_argG*_fkpA(Y95M), C. gl::argR*_argG*_fkpA(Y95S), C. gl::argR*_argG*_fkpA(Y95T), C. gl::argR*_argG*_fkpA(Y95N), C. gl::argR*_argG*_fkpA(Y95C), C. gl::argR*_argG*_fkpA(Y95H), C. gl::argR*_argG*_fkpA(Y95K), C. gl::argR* _argG*_fkpA(Y95D), C. gl::argR*_argG*_fkpA(Y95A), C. gl::argR*_argG*_fkpA(Y95V), C. gl::argR*_argG*_fkpA(Y95L), C. gl::argR*_argG*_fkpA(Y95Q), C. gl::argR*_argG*_fkpA(Y95G), C. gl::argR*_argG*_fkpA(Y95P), and C. gl::argR*_argG*_fkpA(Y95E), respectively.

### Example 2-3. Evaluation of L-Citrulline-Producing Ability

The C. gl::argR*_argG*_fkpA(Y95M), C. gl::argR*_argG*_fkpA(Y95S),
C. gl::argR*_argG*_fkpA(Y95T), C. gl::argR*_argG*_fkpA(Y95N),
C. gl::argR*_argG*_fkpA(Y95C), C. gl::argR*_argG*_fkpA(Y95H),
C. gl::argR*_argG*_fkpA(Y95K), C. gl::argR*_argG*_fkpA(Y95D),
C. gl::argR*_argG*_fkpA(Y95A), C. gl::argR*_argG*_fkpA(Y95V),
C. gl::argR*_argG*_fkpA(Y95L), C. gl::argR*_argG*_fkpA(Y95Q),
C. gl::argR*_argG*_fkpA(Y95G), C. gl::argR*_argG*_fkpA(Y95P), and
C. gl::argR*_argG*_fkpA(Y95E) strains obtained in Example 2-2 and the control C. gl::argR*_argG* were cultured using the following production medium and their L-citrulline-producing abilities were compared. Each strain was inoculated into a 250 ml corner-baffled flask containing 25 ml of the production medium, and cultured at 33°C for 48 hours with shaking at 200 rpm. After the culture was completed, the concentrations of L-citrulline were measured using HPLC, and the results are shown in Table 5 below.

### <Production Medium>

50 g of raw sugar, 30 g of (NH₄)₂SO₄, 1 g of yeast extract, 1.1 g of KH₂PO₄, 1.2 g of MgSO₄·7H₂O, 0.2 g of L-arginine, 1 mg of biotin, 5 mg of thiamine HCl, 5 mg of calcium pantothenate, 15 mg of nicotinamide, 10 mg of MnSO₄, 10 mg of FeSO₄, 0.5 mg of ZnSO₄, 0.5 mg of CuSO₄, 30 g of CaCO₃, pH 7.2 (based on 1 liter of distilled water)

**[Table 5]**

| Strains | Concentration of L-citrulline (g/L) |
|---|---|
| C. gl::argR*_argG* | 3.0 |
| C. gl::argR*_argG*_fkpA(Y95M) | 4.8 |
| C. gl::argR*_argG*_fkpA(Y95S) | 4.7 |
| C. gl::argR*_argG*_fkpA(Y95T) | 4.8 |
| C. gl::argR*_argG*_fkpA(Y95N) | 4.8 |
| C. gl::argR*_argG*_fkpA(Y95C) | 4.7 |
| C. gl::argR*_argG*_fkpA(Y95H) | 4.2 |
| C. gl::argR*_argG*_fkpA(Y95K) | 4.3 |
| C. gl::argR*_argG*_fkpA(Y95D) | 4.4 |
| C. gl::argR*_argG*_fkpA(Y95A) | 4.2 |
| C. gl::argR*_argG*_fkpA(Y95V) | 4.9 |
| C. gl::argR*_argG*_kpA(Y95L) | 4.4 |
| C. gl::argR*_argG*_fkpA(Y95Q) | 4.4 |
| C. gl::argR*_argG*_fkpA(Y95G) | 4.6 |
| C. gl::argR*_argG*_fkpA(Y95P) | 4.4 |
| C. gl::argR*_argG*_fkpA(Y95E) | 4.4 |

As a result, as shown in Table 5 above, all strains, in which the amino acid at position 95 from the N-terminus of prolyl isomerase was substituted from tyrosine to methionine, serine, threonine, asparagine, cysteine, histidine, lysine, aspartic acid, alanine, valine, leucine, glutamine, glycine, proline and glutamic acid, showed increased L-citrulline production and yields, as compared to the control C. gl::argR*_argG*.

### Example 3: Evaluation of L-Glutamine-Producing Ability

### Example 3-1. Determination of Control Group and Preparation of Mutant Prolyl Isomerase Expression Vector

KFCC10680 (Korean Patent No. 10-0048440) was used as a control strain for evaluating L-glutamine-producing ability.

In order to prepare mutant strains, in which a point mutation was introduced into prolyl isomerase based on the KFCC10680 strain, the KFCC10680 strain was transformed with pDCM2-fkpA(Y95V) substituted with a nonpolar amino acid, pDCM2-fkpA(Y95T) substituted with a polar amino acid, and pDCM2-fkpA(Y95D) substituted with a charged amino acid, among pDCM2-fkpA(Y95M), pDCM2-fkpA(Y95S), pDCM2-fkpA(Y95T), pDCM2-fkpA(Y95N), pDCM2-fkpA(Y95C), pDCM2-fkpA(Y95H), pDCM2-fkpA(Y95K), pDCM2-fkpA(Y95D), pDCM2-fkpA(Y95A), pDCM2-fkpA(Y95V), pDCM2-fkpA(Y95L), pDCM2-fkpA(Y95Q), pDCM2-fkpA(Y95G), pDCM2-fkpA(Y95P) and pDCM2-fkpA(Y95E) of Example 1 by electroporation, and then a second crossover process was performed to obtain strains, in which the amino acid at position 95 from the N-terminus of prolyl isomerase was substituted from tyrosine to valine, threonine, and aspartic acid, respectively. The DNA fragments including the *fkpA* gene within the chromosome were amplified by PCR using a primer pair of SEQ ID NOS: 33 and 50 in the genome of the thus-obtained strains. PCR was performed under conditions of denaturation at 95°C for 10 minutes, followed by 30 cycles of denaturation at 95°C for 30 seconds, annealing at 55°C for 30 seconds, and polymerization at 72°C for 2 minutes, and then polymerization at 72°C for 10 minutes. The nucleotide sequences of the amplified gene were analyzed, and as a result, it was confirmed that a mutation was introduced into the nucleotide sequences at positions 283 to 285 downstream from the ORF initiation codon of the *fkpA* gene in each strain (Table 6).

**[Table 6]**

| Amino acids before substitution (nucleotide sequence) | Amino acids after substitution (nucleotide sequence) |
|---|---|
| Tyr(TAC) | Val(GTG) |
| | Thr(ACC) |
| | Asp(GAT) |

Accordingly, mutant strains, in which the amino acid at position 95 from the N-terminus of prolyl isomerase was substituted from tyrosine to valine, threonine, and aspartic acid, were obtained based on the KFCC10680 strain, and the thus-obtained mutant strains were named KFCC10680::fkpA(Y95V), KFCC10680::fkpA(Y95T), and KFCC10680::fkpA(Y95D), respectively.

### Example 3-2. Evaluation of Glutamine-Producing Ability

The KFCC10680::fkpA(Y95V), KFCC10680::fkpA(Y95T), and KFCC10680::fkpA(Y95D) strains obtained in Example 3-1 and the control KFCC10680 were cultured using the following production medium and their L-glutamine-producing abilities were compared. Each strain was inoculated into a 250 ml corner-baffled flask containing 25 ml of the production medium, and cultured at 32°C for 48 hours with shaking at 200 rpm. After the culture was completed, the concentrations of L-glutamine were measured using HPLC, and the results are shown in Table 7 below.

### <Production Medium>

60 g of raw sugar, 30 g of (NH₄)₂SO₄, 1 g of yeast extract, 0.6 g of KH₂PO₄, 0.6 g of MgSO₄·7H₂O, 0.8 mg of biotin, 4 mg of thiamine HCl, 4 mg of calcium pantothenate, 20 mg of nicotinamide, 8 mg of MnSO₄, 8 mg of FeSO₄, 0.4 mg of ZnSO₄, 0.4 mg of CuSO₄, 30 g of CaCO₃, pH 6.8 (based on 1 liter of distilled water)

**[Table 7]**

| Strains | Production of L-Glutamine (g/L) |
|---|---|
| KFCC10680 | 13.2 |
| KFCC10680::fkpA(Y95V) | 16.6 |
| KFCC10680::fkpA(Y95T) | 16.5 |
| KFCC10680::fkpA(Y950) | 15.5 |

As a result, as shown in Table 7 above, all strains, in which the amino acid at position 95 from the N-terminus of prolyl isomerase was substituted from tyrosine to valine, threonine, and aspartic acid, showed increased L-glutamine production and yields, as compared to the control KFCC10680.

### Example 4: Evaluation of L-Ornithine-Producing Ability

### Example 4-1. Preparation of Control Strain

In order to prepare a control strain for the evaluation experiment of L-ornithine-producing ability, a vector was constructed, in which the vector replaces serine at position 55 of a protein sequence of ArgF(ANU33618.1) with a stop codon. The genome of wild-type C. *glutamicum* ATCC 13869 was used as a template, and homologous recombinant A arm and homologous recombinant B arm were amplified using a primer pair of SEQ ID NOS: 61 and 62 and a primer pair of SEQ ID NOS: 63 and 64, respectively. Thereafter, a plasmid was obtained in the same manner as in Example 1, which was named pDCM2-argF(S55*).

Further, in order to prepare a strain with further enhanced L-ornithine-producing ability, a vector was constructed, in which the vector replaces glutamic acid at position 47 of a protein sequence of ArgR(ANU33619.1) with a stop codon. The genome of wild-type C. *glutamicum* ATCC 13869 was used as a template, and homologous recombinant A arm and homologous recombinant B arm were amplified using a primer pair of SEQ ID NOS: 53 and 54 and a primer pair of SEQ ID NOS: 55 and 56, respectively. Thereafter, a plasmid was obtained in the same manner as in Example 1, which was named pDCM2-argR(E47*).

The primer sequences used herein are shown in Table 8 below.

**[Table 8]**

| SEQ ID NO: | Sequence Name | Sequences (5' -> 3') |
|---|---|---|
| 53 | primer 21 | CGGTACCCGGGGATCCCTCGTGCGGAATTCGTGGAG |
| 54 | primer 22 | ATCCAGCAGCAATTCAGACA |
| 55 | primer 23 | CTGAATTGCTGCTGGATTAAGGCATCGATATCACCCA |
| 56 | primer 24 | ATGCCTGCAGGTCGACCCTTCATTTTAAGTTCCTTG |
| 61 | primer 29 | CGGTACCCGGGGATCCTGACCCCAGGCAAGCACGG |
| 62 | primer 30 | GAAGCGAGTACGAGTTTAAGTCTTATC |
| 63 | primer 31 | AAACTCGTACTCGCTTCTCC |
| 64 | primer 32 | ATGCCTGCAGGTCGACCGGCGCCGGCAACCTCGTC |

The wild-type *C*. *glutamicum* ATCC 13869 was transformed with the pDCM2-argF(S55*) vector prepared above by electroporation (Appl. Microbiol.Biotechnol. (1999) 52:541-545), and then a second crossover process was performed to obtain a strain, in which a nucleotide sequence at position 164 of argF was substituted from cytosine (c) to adenine (a) and thus a protein sequence at position 55 was substituted with a stop codon. PCR and sequencing analysis were performed using a primer pair of SEQ ID NOS: 61 and 63 which are capable of amplifying the adjacent region including the position where the corresponding gene was inserted, and the corresponding genetic manipulation was confirmed. The strain thus obtained was named C. gl::argF*.

In order to prepare a strain with further enhanced L-ornithine production in the gl::argF* obtained above, the pDCM2-argR(E47*) vector was used to obtain a strain in the same manner as above. PCR and sequencing analysis were performed using a primer pair of SEQ ID NOS: 53 and 56 which are capable of amplifying the adjacent region including the position where the corresponding gene was inserted, and the corresponding genetic manipulation was confirmed. The strain thus obtained was named C. gl::argR*_argF*.

### Example 4-2. Preparation of Mutant Prolyl Isomerase Expression Vector

In order to prepare mutant strains, in which a point mutation was introduced into prolyl isomerase based on the C. gl::argR*_argF* strain obtained in Example 4-1, the C. gl::argR*_argF* strain was transformed with pDCM2-fkpA(Y95V), pDCM2-fkpA(Y95T), and pDCM2-fkpA(Y95D) of Example 1 by electroporation, and then a second crossover process was performed to obtain strains, in which the amino acid at position 95 from the N-terminus of prolyl isomerase was substituted from tyrosine to valine, threonine, and aspartic acid, respectively. The DNA fragments including the *fkpA* gene within the chromosome were amplified by PCR using a primer pair of SEQ ID NOS: 33 and 50 in the genome of the thus-obtained strains. PCR was performed under conditions of denaturation at 95°C for 10 minutes, followed by 30 cycles of denaturation at 95°C for 30 seconds, annealing at 55°C for 30 seconds, and polymerization at 72°C for 2 minutes, and then polymerization at 72°C for 10 minutes. The nucleotide sequences of the amplified gene were analyzed, and as a result, it was confirmed that a mutation was introduced into the nucleotide sequences at positions 283 to 285 downstream from the ORF initiation codon of the *fkpA* gene in each strain (Table 9).

**[Table 9]**

| Amino acids before substitution (nucleotide sequence) | Amino acids after substitution (nucleotide sequence) |
|---|---|
| Tyr(TAC) | Val(GTG) |
| | Thr(ACC) |
| | Asp(GAT) |

Accordingly, mutant strains, in which the amino acid at position 95 from the N-terminus of prolyl isomerase was substituted from tyrosine to valine, threonine, and aspartic acid, were obtained based on the C. gl::argR*_argF* strain, and the thus-obtained mutant strains were named C. gl::argR*_argF*_fkpA(Y95V), C. gl::argR*_argF*_fkpA(Y95T), and C. gl::argR*_argF*_fkpA(Y95D), respectively.

### Example 4-3. Evaluation of L-Ornithine-Producing Ability

The C. gl::argR*_argF*_fkpA(Y95V), C. gl::argR*_argF*_fkpA(Y95T), and C. gl::argR*_argF*_fkpA(Y95D) strains obtained in Example 4-2, and the control C. gl::argR*_argF* were cultured using the following production medium, and their L-ornithine-producing abilities were compared. Each strain was inoculated into a 250 ml corner-baffled flask containing 25 ml of the production medium, and cultured at 32°C for 48 hours with shaking at 200 rpm. After the culture was completed, the concentrations of L-ornithine were measured using HPLC, and the results are shown in Table 10 below.

### <Production Medium>

50 g of raw sugar, 30 g of (NH₄)₂SO₄, 1 g of yeast extract, 1.1 g of KH₂PO₄, 1.2 g of MgSO₄·7H₂O, 0.2 g of L-arginine, 1 mg of biotin, 5 mg of thiamine HCl, 5 mg of calcium pantothenate, 15 mg of nicotinamide, 10 mg of MnSO₄, 10 mg of FeSO₄, 0.5 mg of ZnSO₄, 0.5 mg of CuSO₄, 30 g of CaCO₃, pH 7.2 (based on 1 liter of distilled water)

**[Table 10]**

| Strains | Production of L-ornithine |
|---|---|
| | (g/L) |
| C. gl::argR*_argF* | 15.7 |
| C. gl::argR*_argF*_fkpA(Y95V) | 16.4 |
| C. gl::argR*_argF*_fkpA(Y95T) | 16.3 |
| C. gl::argR*_argF*_fkpA(Y95D) | 16.1 |

As a result, as shown in Table 10 above, all strains, in which the amino acid at position 95 from the N-terminus of prolyl isomerase was substituted from tyrosine to valine, threonine, and aspartic acid, showed increased L-ornithine production and yields, compared to the control C. gl::argR*_argF*.

### Example 5: Evaluation of L-Arginine-Producing Ability

### Example 5-1. Preparation of Control Group

In order to prepare a control strain for the evaluation experiment of L-arginine-producing ability, a vector was constructed, in which the vector replaces glutamic acid at position 47 of a protein sequence ArgR(ANU33619.1) with a stop codon. The genome of wild-type C. *glutamicum* ATCC 13869 was used as a template, and homologous recombinant A arm and homologous recombinant B arm were amplified using a primer pair of SEQ ID NOS: 53 and 54 and a primer pair of SEQ ID NOS: 55 and 56, respectively. Thereafter, a plasmid was obtained in the same manner as above, which was named pDCM2-argR(E47*).

The primer sequences used herein are shown in Table 11 below.

**[Table 11]**

| SEQ ID NO: | Sequence Name | Sequences (5' -> 3') |
|---|---|---|
| 53 | primer 21 | CGGTACCCGGGGATCCCTCGTGCGGAATTCGTGGAG |
| 54 | primer 22 | ATCCAGCAGCAATTCAGACA |
| 55 | primer 23 | CTGAATTGCTGCTGGATTAAGGCATCGATATCACCCA |
| 56 | primer 24 | ATGCCTGCAGGTCGACCCTTCATTTTAAGTTCCTTG |

The wild-type *C*. *glutamicum* ATCC 13869 was transformed with the pDCM2-argR(E47*) vector prepared above by electroporation (Appl. Microbiol.Biotechnol. (1999) 52:541-545), and then a second crossover process was performed to obtain a strain, in which a nucleotide sequence at position 139 of argR was substituted from guanine (g) to thymine (t) and thus a protein sequence at position 47 was substituted with a stop codon. PCR and sequencing analysis were performed using a primer pair of SEQ ID NOS: 53 and 56 which are capable of amplifying the adjacent region including the position where the corresponding gene was inserted, and the corresponding genetic manipulation was confirmed. The strain thus obtained was named C. gl::argR*.

### Example 5-2. Preparation of Mutant Prolyl Isomerase Expression Vector

In order to prepare mutant strains, in which a point mutation was introduced into prolyl isomerase based on the C. gl::argR* strain obtained in Example 5-1, the C. gl::argR* strain was transformed with pDCM2-fkpA(Y95V), pDCM2-fkpA(Y95T), and pDCM2-fkpA(Y95D) of Example 1 by electroporation, and then a second crossover process was performed to obtain strains, in which the amino acid at position 95 from the N-terminus of prolyl isomerase was substituted from tyrosine to valine, threonine, and aspartic acid, respectively. The DNA fragments, including the *fkpA* gene within the chromosome, were amplified by PCR using a primer pair of SEQ ID NOS: 33 and 50 in the genome of the thus-obtained strains. PCR was performed under conditions of denaturation at 95°C for 10 minutes, followed by 30 cycles of denaturation at 95°C for 30 seconds, annealing at 55°C for 30 seconds, and polymerization at 72°C for 2 minutes, and then polymerization at 72°C for 10 minutes. The nucleotide sequences of the amplified gene were analyzed, and as a result, it was confirmed that a mutation was introduced into the nucleotide sequences at positions 283 to 285 downstream from the ORF initiation codon of the *fkpA* gene in each strain (Table 12).

**[Table 12]**

| Amino acids before substitution (nucleotide sequence) | Amino acids after substitution (nucleotide sequence) |
|---|---|
| Tyr(TAC) | Val(GTG) |
| | Thr(ACC) |
| | Asp(GAT) |

Accordingly, mutant strains, in which the amino acid at position 95 from the N-terminus of prolyl isomerase was substituted from tyrosine to valine, threonine, and aspartic acid, were obtained based on the C. gl::argR* strain, and the thus-obtained mutant strains were named C. gl::argR*_ fkpA(Y95V), C. gl::argR*_fkpA(Y95T), and C. gl::argR*_fkpA(Y95D), respectively.

### Example 5-3. Evaluation of L-Arginine-Producing Ability

The C. gl::argR*_fkpA(Y95V), C. gl::argR*_fkpA(Y95T), and C. gl::argR*_(Y95D) strains obtained in Example 5-2 and the control C. gl::argR* were cultured using the following production medium and their L-arginine-producing abilities were compared. Each strain was inoculated into a 250 ml corner-baffled flask containing 25 ml of the production medium, and cultured at 32°C for 48 hours with shaking at 200 rpm. After the culture was completed, the concentrations of L-arginine were measured using HPLC, and the results are shown in Table 13 below.

### <Production Medium>

50 g of raw sugar, 30 g of (NH₄)₂SO₄, 1 g of yeast extract, 1.1 g of KH₂PO₄, 1.2 g of MgSO₄·7H₂O, 0.2 g of L-arginine, 1 mg of biotin, 5 mg of thiamine HCl, 5 mg of calcium pantothenate, 15 mg of nicotinamide, 10 mg of MnSO₄, 10 mg of FeSO₄, 0.5 mg of ZnSO₄, 0.5 mg of CuSO₄, 30 g of CaCO₃, pH 7.2 (based on 1 liter of distilled water)

**[Table 13]**

| Strains | Production of L-arginine (g/L) |
|---|---|
| C. gl::argR* | 1.3 |
| C. gl::argR*_fkpA(Y95V) | 3.0 |
| C. gl::argR*_fkpA(Y95T) | 3.1 |
| C. gl::argR*_fkpA(Y95D) | 2.7 |

As a result, as shown in Table 13 above, all strains in which the amino acid at position 95 from the N-terminus of prolyl isomerase was substituted from tyrosine to valine, threonine, and aspartic acid showed increased L-arginine production and yields, compared to the control C. gl::argR*.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the present disclosure is defined by the appended claims rather than by the description preceding them, and therefore all changes and modifications that fall within the metes and bounds of the claims, or equivalents of such metes and bounds are therefore intended to be embraced by the claims.

## Claims

1. A mutant prolyl isomerase comprising an amino acid substitution at a position corresponding to position 95 of the amino acid sequence of SEQ ID NO: 1 with another amino acid and having at least 90% sequence identity to the amino acid sequence of SEQ ID NO: 1.

2. The mutant prolyl isomerase of claim 1, wherein another amino acid is any one or more selected from the group consisting of methionine, serine, threonine, asparagine, cysteine, histidine, lysine, aspartic acid, alanine, valine, leucine, glutamine, glycine, proline and glutamic acid.

3. The mutant prolyl isomerase of claim 1, wherein the amino acid at position 95 in the amino acid sequence consisting of SEQ ID NO: 1 is substituted with another amino acid.

4. A polynucleotide encoding the mutant prolyl isomerase of any one of claims 1 to 3.

5. A microorganism comprising any one or more selected from the group consisting of: mutant prolyl isomerase comprising an amino acid substitution at a position corresponding to position 95 of the amino acid sequence of SEQ ID NO: 1 with another amino acid and having at least 90% sequence identity to the amino acid sequence of SEQ ID NO: 1; a polynucleotide encoding the same; and a vector containing the polynucleotide.

6. The microorganism of claim 5, which has an increased production ability of glutamic acid-based amino acids, as compared to a microorganism of the genus Corynebacterium comprising a wild-type prolyl isomerase having an amino acid sequence of SEQ ID NO: 1 or a polynucleotide encoding the same.

7. The microorganism of claim 6, wherein the glutamic acid-based amino acid is any one or more selected from the group consisting of glutamic acid, citrulline, glutamine, ornithine, and arginine.

8. The microorganism of claim 5, wherein the microorganism belongs to the genus Corynebacterium.

9. The microorganism of claim 8, wherein the microorganism of the genus Corynebacterium is Corynebacterium glutamicum.

10. A method for producing glutamic acid-based amino acids, comprising: culturing a microorganism, which comprises mutant prolyl isomerase comprising an amino acid substitution at a position corresponding to position 95 of the amino acid sequence of SEQ ID NO: 1 with another amino acid and having at least 90% sequence identity to the amino acid sequence of SEQ ID NO: 1, or a polypeptide encoding the same, in a medium.

11. The method of claim 9, wherein the glutamic acid-based amino acid is any one or more selected from the group consisting of glutamic acid, citrulline, glutamine, ornithine, and arginine.

12. A composition for producing glutamic acid-based amino acids, comprising: mutant prolyl isomerase comprising an amino acid substitution at a position corresponding to position 95 of the amino acid sequence of SEQ **ID** NO: 1 with another amino acid and having at least 90% sequence identity to the amino acid sequence of SEQ ID NO: 1, a polypeptide encoding the same, a vector containing the polynucleotide, or a microorganism comprising the polynucleotide; a medium on which the microorganism is cultured; or two or more thereof.

13. Use of a mutant prolyl isomerase comprising an amino acid substitution at a position corresponding to position 95 of the amino acid sequence of SEQ **ID** NO: 1 with another amino acid and having at least 90% sequence identity to the amino acid sequence of SEQ ID NO: 1, a polypeptide encoding the same, a vector containing the polynucleotide, or a microorganism comprising the polynucleotide, for the production of glutamic acid-based amino acids.
